(19) **Europäisches Patentamt European Patent Office Office européen des brevets**

(11) **EP 4 671 379 A1**

(12) **EUROPEAN PATENT APPLICATION**
published in accordance with Art. 153(4) EPC

(43) Date of publication:
**31.12.2025 Bulletin 2026/01**

(51) International Patent Classification (IPC):
*C12Q 1/48* (2006.01)     *C12Q 1/686* (2018.01)

(21) Application number: **24760495.2**

(52) Cooperative Patent Classification (CPC):
**C12Q 1/48; C12Q 1/686**

(22) Date of filing: **30.01.2024**

(86) International application number:
**PCT/KR2024/001370**

(87) International publication number:
**WO 2024/177304 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.02.2023 KR 20230023613**

(71) Applicant: **Seegene, Inc.**
**Seoul 05548 (KR)**

(72) Inventors:
• **AHN, Byungjun**
  **Hanam-si, Gyeonggi-do 12955 (KR)**
• **KIM, Myung-Il**
  **Hanam-si, Gyeonggi-do 12916 (KR)**
• **LEE, Jaemin**
  **Yongin-si, Gyeonggi-do 16832 (KR)**

(74) Representative: **Kilger, Ute**
**Boehmert & Boehmert**
**Anwaltspartnerschaft mbB**
**Pettenkoferstrasse 22**
**80336 München (DE)**

(54) **METHOD FOR EVALUATING STATUS OF NUCLEIC ACID POLYMERASE INCLUDED IN PRODUCT OBTAINED FROM PREPARATION OF NUCLEIC ACID POLYMERASE**

(57)     The present invention relates to a method for evaluating the state of a nucleic acid polymerase contained in a product obtained from the preparation process of a nucleic acid polymerase, and a method for obtaining a QC-able nucleic acid polymerase from the product. The present invention can evaluate the state of a nucleic acid polymerase contained in a product obtained from the preparation process of a nucleic acid polymerase by diluting the product obtained from the preparation process of the nucleic acid polymerase to a dilution factor at which specific activity of the nucleic acid polymerase contained in the product shows a substantially constant value.

Fig. 1

100

110 — obtaining the product from the preparation process of the nucleic acid polymerase

120 — diluting the product obtained from the preparation process to a dilution factor at which specific activity of the nucleic acid polymerase contained in the product shows a substantially constant value and measuring the specific activity of the nucleic acid polymerase by using the diluted product

EP 4 671 379 A1

**Description**

**CROSS-REFERENCE TO RELATED APPLICATIONS**

This patent application claims priority from Korean Patent Application No. 2023-0023613, filed on February 22, 2023 in the Korean Intellectual Property Office, the disclosure of which is incorporated herein by reference.

**FIELD OF THE INVENTION**

The present invention relates to a method for evaluating the state of a nucleic acid polymerase contained in a product obtained from a preparation process of the nucleic acid polymerase, or a method for obtaining a QC-able nucleic acid polymerase from the product.

**DESCRIPTION OF THE RELATED ART**

Molecular diagnosis is one of the *in vitro diagnostic* test methods, and it is a method of testing DNA or RNA after amplification using polymerase chain reaction. The polymerase chain reaction continues to be of importance until molecular diagnostic tests for preemptive response to recent COVID-19 infectious diseases. In order to enable such a polymerase chain reaction, it is very important to utilize enzymes that are stable at various temperatures used in the chain reaction and do not lose activity at the same time. Currently, most molecular diagnostic reagents based on polymerization chain reaction utilize polymerases derived from microorganisms that are stable at high temperatures (Hyperthermophiles), and representatively, there is *Taq* DNA polymerase derived from *T. aquaticus* (Chien A., Edgar D. B., Trela J. M. J. Bacteriol. 1976, 127, 1550-1557.).

Among the polymerases used for molecular diagnosis, DNA polymerase is an enzyme that polymerizes DNA using DNA or RNA as a template. In essence, since the main purpose of polymerase is to consume nucleoside triphosphate as a substrate and polymerize it, the activity of the enzyme is measured by how quickly the substrate can be consumed and polymerized, and this is defined as a unit, which is an intrinsic property of the enzyme being measured.

The unit of polymerase means that the unit nucleoside triphosphate is polymerized at a unit temperature in a unit time to become insoluble in acid. For example, 1 unit of *Taq* DNA polymerase from New England Biolabs means that 15 nmol nucleoside triphosphate becomes insoluble in acid *(i.e.,* all polymerized) for 30 minutes at 75°C. Almost similarly, in the case of Bst DNA polymerase, light fragment, 1 unit is defined as 10 nmol nucleoside triphosphate being insoluble in acid for 30 minutes at 65°C.

Although the definition of this unit for companies or products varies slightly depending on the temperature, the amount of substrate, the type of material containing a radioactive isotope, or the like, the reaction rate is generally calculated by measuring the amount of substrate consumed using a radioactive isotope or measuring the amount of insoluble polymer, and the unit is defined through this (Charles C.Richardson, Carl L.Schildkraut, H. Vasken Aposhian, Arthur Kornberg J. Biol. Chem. 1964, 239(1), 222-232.).

The definition of the unit may serve as an analysis method for diagnosis in itself as well as a role as a performance indicator of a molecular diagnostic reagent. Therefore, accurately analyzing the unit measurement of polymerase has become an important issue for several reasons.

Among the unit measurement methods of enzymes, the unit measurement method of enzymes based on radioactive isotopes is a method with high accuracy, but since radioactive isotopes can cause environmental problems and, above all, can affect the health of an experimenter, research on methods for defining units of enzymes without using isotopes has been conducted for decades. Units were measured by changes in the fluorescence values of single-stranded binding proteins (Mark A.Griep, Anal. Biochem., 1995, 232, 180-189.), by using microplate reading of fluorescently labeled nucleotides (Liming Yu, Guolu Hu, Leighton Howells, Biotechniques 2002, 33, 938-941), or by using molecular beacons or electrochemical methods. In addition, an attempt was made to measure the unit using a fluorophore capable of binding to DNA and exhibiting strong fluorescence (H Tveit, T Kristensen, Anal. Biochem., 2001, 289, 96-98.).

Since the aforementioned degree of polymerase activity is an enzyme-specific value, the same amount of polymerase should theoretically represent the same degree of polymerization activity. This is referred to as specific activity. Specific activity in diagnostic enzymes is one of the central indicators for determining the specificity of the enzyme itself, since it can define differences in polymerization activity between enzymes and is used to define the amount of enzyme required for diagnostic products. Therefore, enzyme products used in diagnostic products were measured polymerization activity using radioisotopes and then the U/volume value divided by the volume of enzyme provided in the product were indicated in the products. It is common not to indicate specific activity in the products because it can infer information about the type of enzyme.

It is crucial to ensure that there is a quality issue in the middle of producing enzymes because, if low-purity enzymes are expressed or even if no target protein is discovered, the resulting economic loss is significant. In addition to discovering problems, monitoring of intermediate products is very important because the quality of the final product can also be adjusted consistently if the same degree of intermediate process products are checked each time.

Similar to biopharmaceuticals, the process of producing and purifying enzymes in diagnostic products is also an important situation in which process-by-process analysis is required to maintain a constant state of protein in order to make high-purity products. However, in general, in the case of enzymes mixed with impurities and inactive proteins, it is very difficult to measure or analyze their activity without going through several purification processes.

In addition, in general, in a protein production process, the most important thing is to obtain the desired target protein with high purity. To do this, it is possible to design the process by monitoring how the performance and purity of the protein change at each stage of the process to determine which steps are effective and which steps are not effective. As a result, it is crucial to examine the protein activity at each stage and to analyze its purity.

However, in the related art, whether an enzyme having a desired molecular weight exists in each process step in an indirect manner such as SDS-PAGE, and only the protein concentration (including all impurities) thereof were measured (Fateme Moazen, Ali Rastegari, Sayed Mehdi Hoseini, Mojtaba Panjehpour, Mehran Miroliaei, Hamid Mir, Mohammad Sadeghi, Adv Biomed Res. 2012, 1(2), 82). A method of determining the presence or absence of an enzyme by the Western blot method is also used, but this only checks the presence or absence of an enzyme, and information on performance or purity cannot be determined. In addition, in order to determine the presence and performance of enzymes for diagnosis through PCR, it was not possible to be used for analysis of intermediate products for culture and purification because it had to be in a high purity state after all purification processes in which many impurities that could affect PCR were removed. Therefore, it has been difficult to ascertain the state of the enzyme in culture and purification intermediates by methods known to date.

However, in order to secure the quality of the aforementioned diagnostic enzyme, monitoring of the enzyme state in the culture and purification process is more important than anything else. Since it is not possible to measure the accurate performance and/or purity of the enzyme for each culture purification process by the conventional method, the present inventors have recognized that it is necessary to develop a new method for analyzing intermediate products of culture and purification.

Throughout this application, various patents and publications are referenced and citations are provided in parentheses. The disclosure of these patents and publications in their entities are hereby incorporated by references into this application in order to more fully describe this invention and the state of the art to which this invention pertains.

## SUMMARY OF THE INVENTION

The present inventors have endeavored to develop a method for monitoring the state (specifically, activity and/or purity) of a nucleic acid polymerase in a product obtained in the preparation process of the nucleic acid polymerase. As a result, the present inventors have completed the present invention by confirming that the state of a nucleic acid polymerase included in a product obtained from a preparation process of a nucleic acid polymerase can be evaluated by confirming a dilution factor at which the specific activity of the nucleic acid polymerase included in the product obtained from the preparation process exhibits a substantially constant value as a result of diluting the product obtained from the preparation process of the nucleic acid polymerase.

Accordingly, it is an object of the present invention to provide a method for evaluating the state of a nucleic acid polymerase contained in a product obtained from a preparation process of the nucleic acid polymerase.

It is another object of the present invention to provide a method for obtaining a QC-able nucleic acid polymerase from a product obtained from a preparation process of the nucleic acid polymerase.

Other objects and advantages of the present invention will become apparent from the detailed description to follow taken in conjugation with the appended claims and drawings.

## DETAILED DESCRIPTION OF THIS INVETNION

I. Method for evaluating the state of a nucleic acid polymerase

In an aspect of the present invention, there is provided a method for evaluating the state of a nucleic acid polymerase contained in a product obtained from a preparation process of the nucleic acid polymerase, comprising:

(a) obtaining the product from the preparation process of the nucleic acid polymerase; wherein the product comprises a protein containing the nucleic acid polymerase, and
(b) diluting the product obtained from the preparation process to a dilution factor at which specific activity of the nucleic acid polymerase contained in the product shows a substantially constant value and measuring the specific activity of the nucleic acid polymerase by using the diluted product; wherein the specific activity is measured from intensity of a signal measured by adding the diluted product to a composition for activity assay of a nucleic acid polymerase and performing nucleic acid polymerization reaction, the composition for activity assay of a nucleic acid polymerase comprises a primer, the primer is extended by the nucleic acid polymerase contained in the product under polymerization condition to form an extended duplex, and a detectable signal is provided in a manner dependent on the extended duplex.

The present inventors have endeavored to develop a method for monitoring the state (specifically, activity and/or purity) of a nucleic acid polymerase in a product obtained in the preparation process of the nucleic acid polymerase. As a result, the present inventors have confirmed that the state of a nucleic acid polymerase included in a product obtained from a preparation process of a nucleic acid polymerase can be evaluated by confirming a dilution factor at which the specific activity of the nucleic acid polymerase included in the product obtained from the preparation process exhibits a substantially constant value as a result of diluting the product obtained from the preparation process of the nucleic acid polymerase.

As used herein, the term "state of nucleic acid polymerase" refers to including the activity and/or purity of a nucleic acid polymerase contained in a product obtained from the preparation process of nucleic acid polymerase.

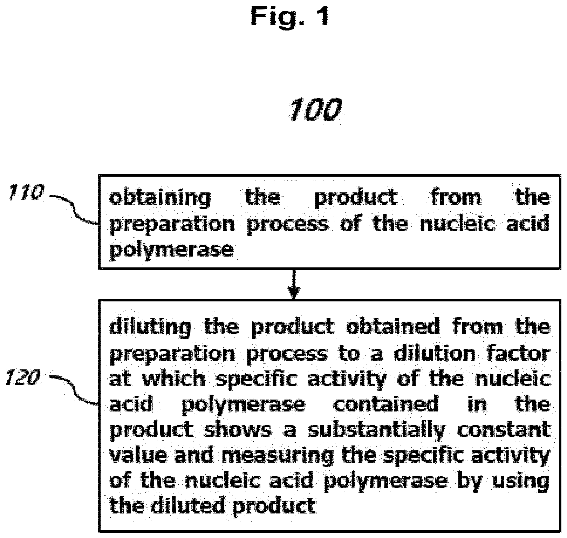

FIG. 1 is a flow chart of a process of performing a method of the present invention according to an embodiment of the present invention. The method of present invention is described with reference to FIG. 1 as follows:

Step (a): obtaining the product from the preparation process of the nucleic acid polymerase (110)

First, the method of the present invention comprises (a) obtaining the product from the preparation process of the nucleic acid polymerase. The product comprises a protein containing the nucleic acid polymerase.

In the present specification, a nucleic acid polymerase is an enzyme polymerizing DNA using DNA or RNA as a template, specifically, a DNA polymerase, and more specifically, a *Taq* DNA polymerase, a KlenTaq DNA polymerase, a Klenow fragment of *E. coli* DNA polymerase I, a DNA polymerase I, a T4 DNA polymerase, a Bst DNA polymerase, a Bsu DNA polymerase, a MMLV reverse transcriptase, a AMV reverse transcriptase or a HIV reverse transcriptase.

According to an embodiment of the present invention, the preparation process of the nucleic acid polymerase is performed by a method including the following steps: (a-1) culturing host cells including an expression construct for a nucleic acid polymerase; and (a-2) purifying the nucleic acid polymerase by applying the resultant of the step (a-1) to a column chromatography.

The preparation process of the nucleic acid polymerase largely includes (a-1) a culture process and (a-2) a purification process.

(a-1) a culture process

As used herein, the term "an expression construct for a nucleic acid polymerase " refers to a nucleic acid molecule including only a minimum element for nucleic acid polymerase expression in a cell.

According to an embodiment, the expression construct for a nucleic acid polymerase is a vector including (i) a nucleic acid polymerase-encoding nucleotide sequence and (ii) a promoter.

As used herein, the term "nucleotide" refers to a deoxyribonucleotide or ribonucleotide present in single-stranded or double-stranded form, and includes analogs of natural nucleotides (Scheit, Nucleotide Analogs, John Wiley, New York(1980); Uhlman and Peyman, Chemical Reviews, 90:543-584(1990)), unless specifically stated otherwise.

As used herein, the term "promoter" refers to an untranslated nucleic acid sequence of the upstream of the coding region, comprising a binding site for the polymerase and having transcriptional initiation activity of the promoter subgene into mRNA.

The expression construct may be constructed through various methods known in the art, and specific methods thereof are disclosed in Sambrook et al., Molecular Cloning, A Laboratory Manual, Cold Spring Harbor Laboratory Press (2001), which is incorporated herein by reference.

When the expression construct is typically constructed as a vector for expression, the vector may be constructed using prokaryotic cells or eukaryotic cells as host cells, and specifically, may be constructed using prokaryotic cells as host cells. When the vector is an expression vector and a prokaryotic cell is used as a host, it generally includes a promoter having strong transcriptional activity *(e.g., a tac* promoter, a *lac* promoter, a *lac*UV5 promoter, a *lpp* promoter, a pLλ promoter, a pRλ promoter, a *rac5* promoter, a *amp* promoter, a *recA* promoter, a SP6 promoter, a *trp* promoter, a T7 promoter, a *phoB* promoter, and the like), a ribosome binding site for initiation of translation, and a transcription/translation termination sequence.

When *E. coli (e.g.,* HB101, BL21, DH5α, etc.) is used as the host cell, the promoter of the *E. coli* tryptophan biosynthetic pathway and the operator site (Yanofsky, C., J. Bacteriol., 158:1018-1024(1984)) and the leftward promoter of phage λ (pL λ promoter, Herskowitz, I. and Hagen, D., Ann. Rev. Genet., 14:399-445 (1980)) may be used as a promoter.

When *Bacillus* bacteria is used as a host cell, promoter of the toxin protein gene of *Bacillus thuringiensis* (Appl. Environ. Microbiol. 64:3932-3938(1998); Mol. Gen. Genet. 250:734-741 (1996)) or any promoter that is expressible in *Bacillus* bacteria can be used as a promoter.

When the vector is prepared for eukaryotic cell expression, the available promoters include, but are not limited to, for example, a CMV promoter, an adenoviral late promoter, a vaccinia virus 7.5K promoter, a SV40 promoter, a *tk* promoter of HSV, a RSV promoter, a EF1 alpha promoter, a metallothionine promoter, a beta-actin promoter, a human IL-2 gene promoter, a promoter of a human IFN gene, a promoter of a human IL-4 gene, a promoter of a human lymphotoxin gene, and a promoter of a human GM-CSF gene.

The vector for eukaryotic cell expression is preferably that a polyadenylation sequence is bonded downstream of the nucleic acid polymerase-encoding nucleotide. The polyadenylated sequence includes a SV40-derived polyadenylated sequence (Schek, N, et al., Mol. Cell Biol. 12:5386-5393(1992)), HIV-1 polyA(Klasens, B. I. F., et al., Nucleic Acids Res. 26:1870-1876(1998)), β-globin polyA(Gil, A., et al, Cell 49:399-406(1987)), HSV TK polyA(Cole, C. N. and T. P. Stacy, Mol. Cell. Biol. 5:2104-2113 (1985)) or polyomavirus polyA (Batt, D. B and G. G. Carmichael, Mol. Cell. Biol. 15:4783-4790 (1995)), but is not limited to.

The expression "a nucleic acid polymerase-encoding nucleotide" as used herein refers to a CDS (coding sequence) nucleotide of a nucleic acid polymerase, which is a target protein or a protein of interest.

The expression construct may have a selectable (or selection) marker for selection. Specifically, the selection marker is a polynucleotide encoding a gene exhibiting antibiotic resistance. The genes exhibiting antibiotic resistance include, for example, resistance genes to ampicillin, gentamicin, carbenicillin, chloramphenicol, streptomycin, kanamycin, geneticin, neomycin, and tetracycline.

More specifically, the vector may be manufactured by manipulating a plasmid (*e.g.*, pDG1661, pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19 series, and the like), phage (*e.g.*, λgt4·λB, λ-Charon, λΔz1, M13, and the like), or virus (*e.g.*, SV40, and the like), and most specifically, may be manufactured by manipulating a plasmid.

Meanwhile, the method of introducing the above-described vector into cells may be performed by various methods known in the art, and examples thereof include microinjection (Capecchi, M.R., Cell, 22:479(1980); and Harland and Weintraub, J. Cell Biol. 101:1094-1099(1985)), calcium phosphate precipitation (Graham, F.L. et al., Virology, 52:456(1973); and Chen and Okayama, Mol. Cell. Biol. 7:2745-2752(1987)), electroporation (Neumann, E. et al., EMBO J., 1:841(1982); and TurKaspa et al., Mol. Cell Biol., 6:716-718(1986)), liposome-mediated transfection (Wong, T.K. et al., Gene, 10:87(1980); Nicolau and Sene, Biochim. Biophys. Acta, 721:185-190(1982); and Nicolau et al., Methods Enzymol., 149:157-176(1987)), DEAE-dextran treatment (Gopal, Mol. Cell Biol., 5:1188-1190(1985)), and gene bombardment (Yang et al., Proc. Natl. Acad. Sci., 87:9568-9572 (1990))

As the host cell, any known host cell capable of stably and continuously expressing the expression construct may be used. The host cell may be a prokaryotic or eukaryotic cell.

When prokaryotic cells are used as the host cells, examples thereof include *E. coli* JM109, *E. coli*BL21, *E. coli* RR1, *E. coli* LE392, *E. coli* B, *E. coli*X 1776, *E. coli* W3110, strains of the genus *Bacillus* such as *Bacillus subtilis, Bacillus churingiensis,* and strains of the enterobacteria such as *Salmonella typhimurium, Serratia marcescens,* and various *Pseudomonas* species. When eukaryotic cells are used as host cells, yeast *(Saccharomyce cerevisiae),* insect cells and human cells *(e.g.,* CHO cell lines (Chinese hamster ovary), W138, BHK, COS-7, 293, HepG2, 3T3, RIN and MDCK cell lines) may be used.

According to an embodiment of the present invention, the host cell is a prokaryotic cell, and most specifically, *E. coli.*

The culture of the transformed host cells may be performed using a medium commonly used in the art. For example, when the transformed host cell is a prokaryotic cell *(e.g., E. coli*), it may be cultured using LB (Luria-Bertani) medium. Various methods of culturing transgenic host cells are well known to those skilled in the art and are disclosed in Sambrook et al., Molecular Cloning, A Laboratory Manual, Cold Spring Harbor Laboratory Press (2001), which is incorporated herein by reference.

According to an embodiment, the step (a-1) includes (a-1-1) crushing the cultured host cells.

The cultured host cells may be crushed by various methods known in the art, and specifically, a crushing method using sonication or glass beads may be used, and more specifically, the cultured host cells may be crushed by ultrasonic crushing.

According to an embodiment of the present invention, the step (a-1) includes (a-1-2) heat-treating the cultured host cells or the lysates obtained by crushing the cultured host cells. More specifically, the heat treatment is performed at 60-120°C for 30-120 minutes.

(a-2) a purification process

As used herein, the term "chromatography" refers to a process of separating a solute of interest in a mixture from other solutes in the mixture by a difference in the rate of movement of each solute through a stationary phase under the influence of a moving phase.

The purification process may be performed using a method of purifying a protein known in the art, for example, the purification process may be performed by filtration using silica gel or celite gel columns, size-exclusion chromatography using liquid column chromatography, ion-exchange chromatography, distribution chromatography, affinity chromatography, or a combination thereof.

According to an embodiment, the step (a-2) includes (a-2-1) purifying the nucleic acid polymerase by applying the resultant of the step to ion-exchange chromatography, and (a-2-2) purifying the nucleic acid polymerase by applying the resultant of the step to affinity chromatography.

Specifically, the step (a-2-1) is carried out by anion exchange column chromatography (more specifically, quaternary amino group-functionalized anion exchange column chromatography) purification from the resultant of the step.

Specifically, for purification by the affinity chromatography, the nucleic acid polymerase used in the present invention is in the form of a fusion protein fused with any one selected from the group consisting of glutathione-S-transferase (GST), maltose binding protein (MBP), FLAG, 6x His (hexahistidine), NusA (N utilization substance A) and Trx (thioredoxin). For example, when a glutathione-S-transferase is fused to a nucleic acid polymerase, glutathione, which is a substrate of the enzyme, may be used, and when 6x His (His-tag) is used, purification may be performed quickly and easily by using a column packed with Ni-NTA resin.

As used herein, the term "a product obtained from preparation process" refers to a solution obtained by aliquoting a certain amount at each stage of culture and purification in producing a nucleic acid polymerase.

In the present specification, the product in the preparation process may be largely a culture product and a purification product, and more specifically, a heat treatment product, an ion exchange column purification product, and a metal bond column purification product.

The product obtained from the preparation process includes a protein comprising nucleic acid polymerase. The proteins included in the product include all proteins included in the host cell other than the target protein to be expressed and produced. Specifically, the protein may include not only nucleic acid polymerase having activity but also impurities including an inactive protein.

According to an embodiment of the present invention, the method further comprises, after step (a), (a-3) determining mass of the protein contained in the product obtained from the preparation process from (i) volume of the product obtained from the preparation process of step (a) and (ii) concentration of the protein in the product.

In the present embodiment, the reason for determining the mass of the protein contained in the product obtained from the preparation process of a nucleic acid polymerase is not only to determine the specific activity of nucleic acid polymerase contained in a product obtained from the preparation process, but also to determine the amount of the nucleic acid polymerase having activity among the proteins contained in the product obtained from the preparation process.

In the present embodiment, the concentration of the protein contained in the product obtained from the preparation process of a nucleic acid polymerase may be measured by various methods known in the art, for example, the concentration of the protein may be measured using Bradford method, BCA method, or A280 absorption.

Step (b): measuring the specific activity of the nucleic acid polymerase **(120)**

Finally, the method of the present invention comprises (b) diluting the product obtained from the preparation process to a dilution factor at which specific activity of the nucleic acid polymerase contained in the product shows a substantially constant value and measuring the specific activity of the nucleic acid polymerase by using the diluted product. The specific activity is measured from intensity of a signal measured by adding the diluted product to a composition for activity assay of a nucleic acid polymerase and performing nucleic acid polymerization reaction, the composition for activity assay of a nucleic acid polymerase comprises a primer, the primer is extended by the nucleic acid polymerase contained in the product under polymerization condition to form an extended duplex, and a detectable signal is provided in a manner dependent on the extended duplex.

The term "activity" used herein with reference to nucleic acid polymerase refers to the polymerization activity of nucleic acid polymerase, and the term "specific activity" refers to the polymerization activity of nucleic acid polymerase per unit mass of nucleic acid polymerase.

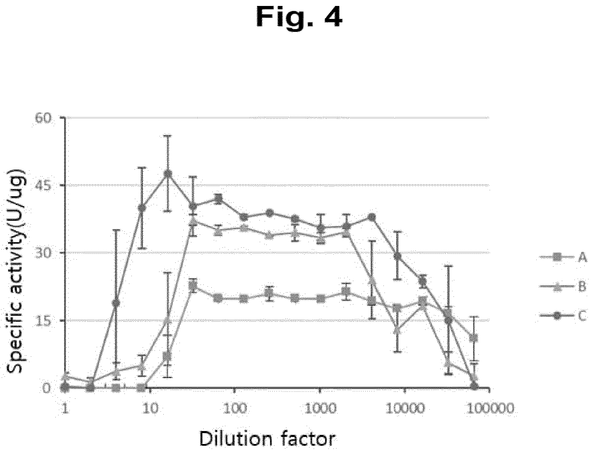

The product obtained from the preparation process of a nucleic acid polymerase is usually obtained in the form of an eluate using the solution as it is without exchange of the base (or blank) solution, and in general, a salt or the like, which is a substance that may interfere with the measurement of polymerization activity in this solution, is given in a high state. If the polymerase activity is measured immediately, the probability of separation of the template DNA to which the primer is attached increases due to the high salt concentration, resulting in an overall signal reduction and calculating the fluorescence value of the graph that cannot be measured. In addition, since the products obtained from the preparation process all have different eluate base (or blank) solutions, the effect on the measurement of enzyme activity may be different, and thus the present inventors confirmed through the dilution of the product obtained from the preparation process of a nucleic acid polymerase whether a constant specific activity value may be measured regardless of the type of eluate, and through this, it is possible to measure the specific activity of the product obtained from the preparation process (specifically, a purified intermediate product) by applying the same dilution factor to the product from any preparation process (specifically, any purified eluate), and this is the biggest feature of the present invention (FIG. 4).

Through these interesting findings, in the case of a product obtained from the preparation process, the specific activity of the nucleic acid polymerase contained in a product obtained from the preparation process may be measured by diluting the product with a dilution factor in which the specific activity of the nucleic acid polymerase contained in a product obtained from the preparation process exhibits a substantially constant value.

In the present specification, "the specific activity of the nucleic acid polymerase contained in the product represents a substantially constant value" means that the specific activity value of the nucleic acid polymerase contained in the product according to the dilution factor of the product in the preparation process is maintained within the range of $\pm 10$ U/ug (specifically, $\pm 7$ U/ug, more specifically, $\pm 5$ U/ug). For example, when the specific activity value is 20 U/ug when diluted 100 times, and when the specific activity value is 23 U/ug or 17 U/ug when diluted 200 times, the specific activity value may be said to be substantially constant, but when the specific activity value is 20 U/ug when diluted 100 times, and when the specific activity value is 32 U/ug or 9 U/ug when diluted 200 times, the specific activity value may not be said to be substantially constant.

According to an embodiment of the present invention, the dilution of step (b) is performed within a range of a dilution factor in which the specific activity of the nucleic acid polymerase contained in the product represents substantially constant value.

According to an embodiment, the dilution of step (b) is performed within a dilution range of 60 to 2000 times.

Through FIG. 4, the present inventors have found that a constant specific activity value can be measured regardless of the type of eluate and the amount of impurities in a dilution range of 60 to 2000 times of the product obtained from the preparation process.

In the present embodiment, when the dilution factor is less than 60 times, there is a disadvantage in that the measurement of the polymerization activity of the nucleic acid polymerase is hindered due to the impurities contained in the product obtained from the preparation process, and when the dilution factor is more than 2000 times, there is a problem in that the polymerization reaction of the nucleic acid polymerase is not sufficiently generated due to excessive dilution, and the intensity of the signal cannot be detected, and thus the activity of the nucleic acid polymerase cannot be monitored.

According to an embodiment of the present invention, the product is at least one product selected from the group consisting of a host cell lysate, a heat treatment product of host cell lysate, and a resultant of column chromatography.

The resultant of column chromatography refers to a product obtained by purifying a product obtained by heat-treating the host cell lysate by a column chromatography purification method.

Since the host cell lysate, the heat treatment product of host cell lysate, and the resultant of column chromatography are the same as the product obtained from the preparation process, the common descriptions between them are omitted in order to avoid undue redundancy leading to the complexity of the present specification.

According to an embodiment, the dilution in step (b) is performed using a buffer, and more specifically, the buffer includes Tris, Cl⁻, and $Mg^{2+}$ and satisfies pH 7 to pH 9.

In this embodiment, the buffer used to dilute the product obtained from the preparation process is a buffer used to provide an optimal pH environment and to prevent rapid changes in pH due to temperature changes and chemical action, and may include, for example, Tris-HCl. The buffer may include a salt for stabilization, the salt may be a salt of magnesium or potassium, for example $MgCl_2$, $MgSO_4$ or KCl. The salt may be added at various concentrations known in the art.

According to an embodiment of the present invention, the concentration of the protein contained in the diluted product in step (b) is 0.0005 mg/ml to 0.0125 mg/ml.

Through the above-described dilution in the range of 60 to 2000 times, the concentration of the protein contained in the diluted product is 0.0005 mg/ml to 0.0125 mg/ml, and it was confirmed that the specific activity value of nucleic acid polymerase contained in a product obtained from the preparation process of a nucleic acid polymerase is constantly measured in the concentration range of the protein, regardless of the type of eluate and the ratio of impurities (FIG. 4).

According to an embodiment, the measurement of the specific activity in step (b) is carried out by a method comprising the following steps: (b-1) diluting the product obtained from the preparation process of step (a) to a dilution factor at which specific activity of the nucleic acid polymerase contained in the product shows a substantially constant value and measuring activity of the nucleic acid polymerase by using the diluted product; wherein the activity is measured from intensity of a signal measured by adding the diluted product to a composition for activity assay of a nucleic acid polymerase and performing nucleic acid polymerization reaction, the composition for activity assay of a nucleic acid polymerase comprises a primer, the primer is extended by the nucleic acid polymerase contained in the product under polymerization condition to form an extended duplex, and a detectable signal is provided in a manner dependent on the extended duplex, and (b-2) providing the activity of the nucleic acid polymerase contained in the product measured in step (b-1) relative to the mass of the protein contained in the product as the specific activity of the nucleic acid polymerase contained in the product obtained from the preparation process.

In order to measure the specific activity of nucleic acid polymerase contained in a product obtained from the preparation process, the activity of the nucleic acid polymerase contained in the product should be measured first.

In the present specification, measuring the activity of nucleic acid polymerase contained in a product obtained from the preparation process is measuring the polymerization activity of nucleic acid polymerase contained in a product obtained from the preparation process, which means quantifying the degree of polymerization reaction through the amount of synthesized nucleic acid at a specific temperature and unit time.

The activity of nucleic acid polymerase contained in a product obtained from the preparation process is measured from the intensity of the signal measured by adding the diluted product to the composition for activity assay of the nucleic acid polymerase and performing a nucleic acid polymerization reaction.

According to an embodiment of the present invention, in step (b) or (b-1), the addition of the diluted product to a composition for activity assay of a nucleic acid polymerase is performed by adding the diluted product to a composition for activity assay of a nucleic acid polymerase by one dilution factor or by each dilution factor within the range of the dilution factor. For example, when the range of the dilution factor is 60 to 2000 times, the product obtained from the preparation process diluted 200 times in the dilution factor range may be added to the composition for activity assay of nucleic acid polymerase, or the products obtained from the preparation process diluted 100 times, 200 times, 300 times, 400 times, and 500 times in the dilution factor range may be added to the composition for activity assay of nucleic acid polymerase, respectively.

The composition for activity assay of a nucleic acid polymerase comprises a primer, the primer is extended by the nucleic acid polymerase contained in the product under polymerization condition to form an extended duplex, and a detectable signal is provided in a manner dependent on the extended duplex.

As used herein, the term "primer" refers to an oligonucleotide that can act as an initiation of synthesis under conditions in which synthesis of a primer extension product complementary to a nucleic acid strand (template) is induced, *i.e.,* the presence of a nucleotide and a polymerization agent such as DNA polymerase, and suitable temperature and pH. In particular, the primers are single-stranded deoxyribonucleotide molecules. Primers used in the present invention may include naturally occurring dNMPs (*i.e.,* dAMP, dGMP, dCMP and dTMP), modified nucleotides, or non-natural nucleotides. In addition, a typical primer may include a ribonucleotide.

The primer must be long enough to prime the synthesis of the extension product in the presence of a polymerization agent. A suitable length of the primer is determined according to a plurality of factors including, for example, temperature, application field, and source of the primer. As used herein, the term "annealing" or "priming" refers to the apposition of an oligodeoxynucleotide or a nucleic acid to a template nucleic acid, the apposition causes polymerase to polymerize the nucleotide to form a nucleic acid molecule complementary to the template nucleic acid or a portion thereof.

The length of the primer may be 7-150 nucleotides, 10-120 nucleotides, 12-100 nucleotides, 14-80 nucleotides, 16-60 nucleotides, 18-40 nucleotides, 20-35 nucleotides, or 20-30 nucleotides.

According to an embodiment, the composition for activity assay of a nucleic acid polymerase further comprises (i) a template, (ii) a label, (iii) dNTP and (iv) a buffer.

(i) Template

In the polymerization reaction of the present invention, a nucleic acid polymerase polymerizes a nucleotide into a template nucleic acid to form a nucleic acid molecule complementary to the template nucleic acid, thereby forming an extended duplex.

Specifically, a template may be generally used by using M13mp18 DNA, which is a single-stranded DNA, or by synthesizing a template DNA to which a primer is connected, but is not limited thereto.

As used herein, the term "extension duplex or extended duplex" refers to a duplex formed by an extension reaction in which a primer hybridized to a template is extended by a template-dependent nucleic acid polymerase.

As used herein, the term "hybridization" means that complementary single-stranded nucleic acids form a double-stranded nucleic acid. Hybridization may occur when the complementarity between two nucleic acid strands is perfect match or even when some mismatch bases are present. The degree of complementarity required for hybridization may vary depending on hybridization conditions, and may be particularly controlled by temperature.

Hybridization of the primer with the template may be performed under suitable hybridization conditions generally determined by optimization procedures. Conditions such as temperature, concentration of components, number of hybridizations and washes, buffer components and their pH and ionic strength may vary depending on various factors including the length of the primer and the GC content. For example, when a relatively short primer is used, it is preferable to select a low stringent condition. Detailed conditions for hybridization can be found in Joseph Sambrook et al., Molecular Cloning, A Laboratory Manual, Cold Spring Harbor Laboratory Press, Cold Spring Harbor, N.Y. (2001); and M.L.M. Anderson, Nucleic Acid Hybridization, Springer-Verlag New York Inc. N.Y. (1999).

The terms "annealing" and "hybridization" are not different and are used interchangeably herein.

The primer has a hybridization nucleotide sequence complementary to the template.

As used herein, the term "complementary" means that the primer is sufficiently complementary to selectively hybridize to the template under predetermined annealing conditions or stringent conditions, and encompasses both "substantially complementary" and "completely complementary", and specifically means that the primer is completely complementary.

The term "non-complementary" means that the primer is sufficiently non-complementary not to be selectively hybridized to the template under predetermined annealing conditions or stringent conditions, encompassing both "substantially non-complementary" and "completely non-complementary", and in particular, means that the primer is completely non-complementary.

(ii) Label

The composition for activity assay of the nucleic acid polymerase may comprise a label to provide a detectable signal in a dependent manner of the extended duplex.

The expression "a detectable signal is provided in a manner dependent on the extended duplex" used herein means that the detectable signal is provided directly or indirectly from the presence of the extended duplex, and specifically the detectable signal may be provided from the extended duplex itself.

More specifically, in the present invention, an intercalating label that provides a signal indicative of the degree of formation of an extended duplex by a nucleic acid polymerase may be used, the intercalating label is selected from the group consisting of SYBR™ Green I, PO-PRO™-1, BO-PRO™-1, SYTO™ 43, SYTO™ 44, SYTO™ 45, SYTOX™ Blue, POPO™-1, POPO™-3, BOBO™-1, BOBO™-3, LO-PRO™-1, JO-PRO™-1, YO-PRO™ 1, TO-PRO™1, SYTO™11, SYTO™ 13, SYTO™15, SYTO™16, SYTO™20, SYTO™23, TOTO™-3, YOYO™3, GelStar™, thiazole orange, PicoGreen®, Eva-Green® and LAM Fluorescent Dye.

The intercalating label specifically intervenes within a double-stranded nucleic acid molecule and generates a signal. By the formation of the extended duplex, the fluorescence intensity from a single fluorescent label is increased. Therefore, it is possible to detect a signal change that increases when the extended duplex is formed in real time.

(iii) dNTP

The composition for activity assay of a nucleic acid polymerase may contain dNTP, which is the building block of DNA or RNA being synthesized. The dNTP comprises a mixture of four deoxyribonucleotides, *i.e.,* dATP, dCTP, dGTP and dTTP (or dUTP).

(iv) Buffer

The composition for activity assay of a nucleic acid polymerase may include a buffer to provide a pH environment for optimal activity of the nucleic acid polymerase and to prevent rapid changes in pH due to temperature changes and chemical action during the polymerization reaction. Examples of buffers include Tris-HCl.

According to another embodiment of the present invention, The composition for activity assay of a nucleic acid polymerase further comprises (v) salt and (vi) additives.

(v) Salt

The composition for activity assay of a nucleic acid polymerase may comprise a salt to help stabilize the activity of the nucleic acid polymerase. The salt may be a salt of magnesium or potassium, for example $MgCl_2$, $MgSO_4$ or KCl. The salt may be added at various concentrations known in the art.

(vi) Additives

The composition for activity assay of a nucleic acid polymerase may include a variety of additives to stabilize the nucleic acid polymerase. Examples of such additives include BSA, DMSO, betaine, KCl, non-ionic surfactants (e.g., Tween 20, Tritox X-100).

The nucleic acid polymerization reaction may include isothermal polymerization reaction, PCR, quantitative real-time PCR (Real time PCR, qPCR), melting curve analysis, high resolution melting analysis, sequencing, quantitative fluorescent PCR, multiplex PCR, digital PCR (dPCR), whole genome amplification (WGA), or reverse transcription PCR.

According to an embodiment, the nucleic acid polymerization reaction is performed at 35°C to 75°C for 30 to 60 minutes.

As used herein, the term "polymerization conditions" refers to conditions of the presence of components necessary for polymerization, and suitable temperature and pH. The term "polymerization conditions" as used herein refers to conditions for separate steps involved in a polymerization reaction, such as annealing/hybridization of a primer and extension of the primer. These conditions may be dependent on a variety of factors, including the length of the primer and the GC content, as well as the length and GC content of the hybridization site. Detailed conditions can be found in Joseph Sambrook et al., Molecular Cloning, A Laboratory Manual, Cold Spring Harbor Laboratory Press, Cold Spring Harbor, N.Y. (2001); and M.L.M. Anderson, Nucleic Acid Hybridization, Springer-Verlag New York Inc. N.Y. (1999).

According to a more specific embodiment of the present invention, the activity of the nucleic acid polymerase in step (b-1) is provided as a slope measured in a range in which the intensity of a signal increases as a linear function depending on the concentration of the protein contained in the product obtained from the preparation process.

Specifically, the activity of nucleic acid polymerase contained in a product obtained from the preparation process may be provided by measuring the slope (differential value of time-signal intensity) of the first function portion with respect to the signal intensity over time for each concentration of protein contained in the product obtained from the preparation process, and calculating the slope of the portion where the differential value of time-signal intensity becomes the first function according to the concentration of protein contained in the product obtained from the preparation process.

According to a more specific embodiment, the nucleic acid polymerase is *Taq* DNA polymerase or KlenTaq DNA polymerase, and the activity of the nucleic acid polymerase in step (b-1) is provided as the amount of a nucleic acid polymerase determined by substituting the intensity of a signal into the following general formula 1:

**General formula 1** 1 unit (enzyme activity unit) = the amount of nucleic acid polymerase required to represent intensity of a signal showing polymerization by consuming $4.4 \pm 0.56$ nmole of dNTP for 30 minutes at 72°C.  ⟨General formula 1⟩

According to a more specific embodiment, the nucleic acid polymerase is *Taq* DNA polymerase or KlenTaq DNA polymerase, and the activity of the nucleic acid polymerase in step (b-1) is provided as the unit number of a nucleic acid polymerase determined by substituting the intensity of a signal into the following general formula 1:

**General formula 1** 1 unit (enzyme activity unit) = the amount of nucleic acid polymerase required to represent intensity of a signal showing polymerization by consuming $4.4 \pm 0.56$ nmole of dNTP for 30 minutes at 72°C.  ⟨General formula 1⟩

According to an embodiment of the present invention, the method further comprises, after step (b), the following steps:

(b-3) providing specific activity of a reference nucleic acid polymerase; and
(b-4) providing the specific activity of the nucleic acid polymerase provided in step (b) relative to the specific activity of the reference nucleic acid polymerase in step (b-3) as purity of the nucleic acid polymerase contained in the product obtained from the preparation process.

According to an embodiment, the reference nucleic acid polymerase is a nucleic acid polymerase whose activity and purity are known. Specifically, the reference nucleic acid polymerase is a nucleic acid polymerase whose activity and purity are known as measured based on radioactive isotopes, and more specifically, it is selected from the group consisting of commercially available *Taq* DNA polymerase from NEB, *Taq* DNA polymerase from Roche, *Taq* DNA polymerase from Qiagen, KlenTaq 1 from DNA Polymerase Technology, and Bst DNA polymerase large fragment from NEB.

According to the present embodiment, the specific activity of the reference nucleic acid polymerase of which the purity is known and the specific activity of nucleic acid polymerase contained in a product obtained from the preparation process of which the purity is unknown may be compared to calculate the purity of nucleic acid polymerase contained in a product obtained from the preparation process.

According to an embodiment of the present invention, the method further comprises (b-5) determining the amount of the nucleic acid polymerase with activity contained in the product obtained from the preparation process from the mass of the protein contained in the product and the purity of the nucleic acid polymerase contained in the product provided in step (b-4).

According to the present embodiment, when the purity of nucleic acid polymerase contained in a product obtained from the preparation process is calculated, the amount of the nucleic acid polymerase in the protein contained in the product obtained from the preparation process may be determined.

According to a more specific embodiment, the measurement of the specific activity of the reference nucleic acid polymerase in step (b-3) is carried out by a method comprising the following steps:

(b-3-1) adding the reference nucleic acid polymerase to a composition for activity assay of a nucleic acid polymerase and performing a nucleic acid polymerization reaction to detect a signal; wherein the composition for activity assay of a nucleic acid polymerase comprises a primer, the primer is extended by the reference nucleic acid polymerase under polymerization conditions to form an extended duplex, and a detectable signal is provided in a manner dependent on the extended duplex;

(b-3-2) providing the activity of the reference nucleic acid polymerase from the intensity of the signal; and

(b-3-3) providing the activity of the reference nucleic acid polymerase relative to the mass of the reference nucleic acid polymerase as the specific activity of the reference nucleic acid polymerase.

Since the measurement of the specific activity of the reference nucleic acid polymerase is the same as the measurement of the specific activity of the nucleic acid polymerase included in the product in the preparation process described above, the common descriptions between them are omitted in order to avoid undue redundancy leading to the complexity of the present specification.

According to an embodiment of the present invention, the method further includes the following steps after step (b):

(b-5) obtaining a product from a preparation process different from the preparation process of the nucleic acid polymerase of step (a); wherein the product comprises a protein containing the nucleic acid polymerase,

(b-6) performing step (b) using the product obtained from the preparation process of step (b-5) to provide the specific activity of nucleic acid polymerase contained in a product obtained from the preparation process of step (b-5); and

(b-7) comparing the specific activity of nucleic acid polymerase contained in a product obtained from the preparation process of step (a) with the activity of nucleic acid polymerase contained in a product obtained from the preparation process of step (b-6).

The product obtained from the preparation process of step (a) and the product obtained from the preparation process of step (b-5) (*i.e.,* the product obtained from the preparation process other than the preparation process of the nucleic acid polymerase of step (a)) may be, for example, the product obtained from the culture process and the product obtained from the purification process in the same production process of the nucleic acid polymerase, or the product obtained from a purification process of one production process and a product obtained from a purification process of the other production process among two different production processes of the nucleic acid polymerase.

When a nucleic acid polymerase having a desired specific activity is obtained with high purity, a process may be designed by monitoring how the state (specific activity and/or purity) of the nucleic acid polymerase contained in the product in each process of the same production process of the nucleic acid polymerase (*e.g.*, culture process and purification process, or purification process 1 and purification process 2) or in the same process of the process of the other two production processes of the nucleic acid polymerase (*e.g.,* metal-linked column purification process and metal-linked column purification process introducing additional column washing process) changes, and determining which process is effective and which process is not effective.

II. Method for obtaining QC-able nucleic acid polymerase

In another aspect of the present invention, there is provided a method for obtaining a QC-able nucleic acid polymerase from a product obtained from a preparation process of the nucleic acid polymerase, comprising:

(a) obtaining the product from the preparation process of the nucleic acid polymerase; wherein the product comprises a protein containing the nucleic acid polymerase,

(b) diluting the product obtained from the preparation process to a dilution factor at which specific activity of the nucleic acid polymerase contained in the product shows a substantially constant value and measuring the specific activity of the nucleic acid polymerase by using the diluted product; wherein the specific activity is measured from intensity of a signal measured by adding the diluted product to a composition for activity assay of a nucleic acid polymerase and performing nucleic acid polymerization reaction, the composition for activity assay of a nucleic acid polymerase comprises a primer, the primer is extended by the nucleic acid polymerase contained in the product under polymerization condition to form an extended duplex, and a detectable signal is provided in a manner dependent on the extended duplex, and

(c) providing the nucleic acid polymerase in which the specific activity of the nucleic acid polymerase contained in the product obtained from the preparation process has been measured.

The present invention relates to a method for obtaining a QC-able nucleic acid polymerase by using a method for evaluating the state of a nucleic acid polymerase in a product obtained from the preparation process of a nucleic acid polymerase of the present invention described above. Therefore, the common descriptions between them are omitted in order to avoid undue redundancy leading to the complexity of the present specification.

As used herein, the expression "a method for obtaining QC-able nucleic acid polymerase " refers to a method for producing a nucleic acid polymerase that enables quality control (QC) of a final purified product, a nucleic acid polymerase, by evaluating and monitoring the state (activity and/or purity) of the nucleic acid polymerase in a product obtained from the preparation process of the nucleic acid polymerase.

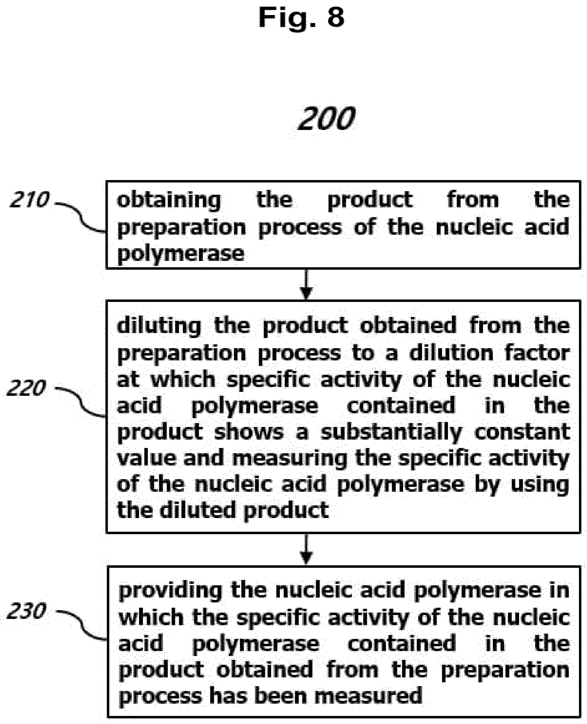

FIG. 8 is a flow chart of a process of performing a method of the present invention according to an embodiment of the present invention. The method of present invention is described with reference to FIG. 8 as follows:

Step (a): obtaining the product from the preparation process of the nucleic acid polymerase **(210)**

First, the method of the present invention comprises (a) obtaining the product from the preparation process of the nucleic acid polymerase. The product comprises a protein containing the nucleic acid polymerase.

According to an embodiment of the present invention, the nucleic acid polymerase is a DNA polymerase.

According to a more specific embodiment, the DNA polymerase is *Taq* DNA polymerase, KlenTaq DNA polymerase, Klenow fragment of *E. coli* DNA polymerase I, DNA polymerase I, T4 DNA polymerase, Bst DNA polymerase, Bsu DNA polymerase, MMLV reverse transcriptase, AMV reverse transcriptase or HIV reverse transcriptase.

According to an embodiment of the present invention, the preparation process of the nucleic acid polymerase is carried out by a method including the following steps: (a-1) culturing host cells including an expression construct for a nucleic acid polymerase; and (a-2) purifying the nucleic acid polymerase by applying the resultant of the step (a-1) to a column chromatography.

According to an embodiment, the method further comprises, after step (a), (a-3) determining mass of the protein contained in the product obtained from the preparation process from (i) volume of the product obtained from the preparation process of step (a) and (ii) concentration of the protein in the product.

Step (b): measuring the specific activity of the nucleic acid polymerase (220)

The method of the present invention then comprises (b) diluting the product obtained from the preparation process to a dilution factor at which specific activity of the nucleic acid polymerase contained in the product shows a substantially constant value and measuring the specific activity of the nucleic acid polymerase by using the diluted product. The specific activity is measured from intensity of a signal measured by adding the diluted product to a composition for activity assay of a nucleic acid polymerase and performing nucleic acid polymerization reaction, the composition for activity assay of a nucleic acid polymerase comprises a primer, the primer is extended by the nucleic acid polymerase contained in the product under polymerization condition to form an extended duplex, and a detectable signal is provided in a manner dependent on the extended duplex.

According to an embodiment of the present invention, the dilution of step (b) is performed within a range of a dilution factor in which the specific activity of the nucleic acid polymerase contained in the product shows substantially constant value.

According to an embodiment, the dilution in step (b) is performed within a dilution range of 60 to 2000 times.

According to an embodiment of the present invention, the product is at least one product selected from the group consisting of a host cell lysate, a heat treatment product of host cell lysate, and a resultant of column chromatography.

According to an embodiment, the dilution in step (b) is performed using a buffer.

According to a more specific embodiment of the present invention, the buffer includes Tris, Cl-, and $Mg^{2+}$ and satisfies pH 7 to pH 9.

According to an embodiment, the concentration of the protein contained in the diluted product in step (b) is 0.0005 mg/ml to 0.0125 mg/ml.

According to an embodiment of the present invention, the measurement of the specific activity in step (b) is carried out by a method comprising the following steps:

(b-1) diluting the product obtained from the preparation process of step (a) to a dilution factor at which specific activity of the nucleic acid polymerase contained in the product shows a substantially constant value and measuring activity of the nucleic acid polymerase by using the diluted product; wherein the activity is measured from intensity of a signal measured by adding the diluted product to a composition for activity assay of a nucleic acid polymerase and performing nucleic acid polymerization reaction, the composition for activity assay of a nucleic acid polymerase comprises a primer, the primer is extended by the nucleic acid polymerase contained in the product under polymerization condition to form an extended duplex, and a detectable signal is provided in a manner dependent on the extended duplex, and

(b-2) providing the activity of the nucleic acid polymerase contained in the product measured in step (b-1) relative to the mass of the protein contained in the product as the specific activity of the nucleic acid polymerase contained in the product obtained from the preparation process.

According to an embodiment, in step (b) or (b-1), the addition of the diluted product to a composition for activity assay of a nucleic acid polymerase is performed by adding the diluted product to a composition for activity assay of a nucleic acid polymerase by one dilution factor or by each dilution factor within the range of the dilution factor.

According to an embodiment of the present invention, the composition for activity assay of a nucleic acid polymerase further comprises (i) a template, (ii) a label, (iii) dNTP and (iv) a buffer.

According to a more specific embodiment, the label is an intercalating label.

According to a more specific embodiment of the present invention, the intercalating label is selected from the group consisting of SYBR™ Green I, PO-PRO™-1, BO-PRO™-1, SYTO™ 43, SYTO™ 44, SYTO™ 45, SYTOX™ Blue, POPO™-1, POPO™-3, BOBO™-1, BOBO™-3, LO-PRO™-1, JO-PRO™-1, YO-PRO™ 1, TO-PRO™1, SYTO™11, SYTO™13, SYTO™15, SYTO™16, SYTO™20, SYTO™23, TOTO™-3, YOYO™3, GelStar™, thiazole orange, PicoGreen®, EvaGreen® and LAM Fluorescent Dye.

According to another embodiment, the composition for activity assay of a nucleic acid polymerase further comprises (v) salt and (vi) additives.

According to an embodiment of the present invention, the nucleic acid polymerization reaction is performed at 35°C to 75°C for 30 to 60 minutes.

According to a more specific embodiment, the activity of the nucleic acid polymerase in step (b-1) is provided as a slope measured in a range in which the intensity of a signal increases as a linear function depending on the concentration of the protein contained in the product obtained from the preparation process.

According to a more specific embodiment of the present invention, the nucleic acid polymerase is *Taq* DNA polymerase or KlenTaq DNA polymerase, and the activity of the nucleic acid polymerase in step (b-1) is provided as the amount of a nucleic acid polymerase determined by substituting the intensity of a signal into the following general formula 1:

**General formula 1** 1 unit (enzyme activity unit) = the amount of nucleic acid polymerase required to represent intensity of a signal showing polymerization by consuming 4.4±0.56 nmole of dNTP for 30 minutes at 72°C.  General formula 1

According to a more specific embodiment, the nucleic acid polymerase is *Taq* DNA polymerase or KlenTaq DNA polymerase, and the activity of the nucleic acid polymerase in step (b-1) is provided as the unit number of a nucleic acid polymerase determined by substituting the intensity of a signal into the following general formula 1:

**General formula 1** 1 unit (enzyme activity unit) = the amount of nucleic acid polymerase required to represent intensity of a signal showing polymerization by consuming 4.4±0.56 nmole of dNTP for 30 minutes at 72°C.  General formula 1

According to an embodiment of the present invention, the method further comprises, after step (b), the following steps:

(b-3) providing specific activity of a reference nucleic acid polymerase; and
(b-4) providing the specific activity of the nucleic acid polymerase provided in step (b) relative to the specific activity of the reference nucleic acid polymerase in step (b-3) as purity of the nucleic acid polymerase contained in the product obtained from the preparation process.

According to an embodiment, the reference nucleic acid polymerase is a nucleic acid polymerase with known activity and purity.

According to a more specific embodiment of the present invention, the reference nucleic acid polymerase is selected from the group consisting of *Taq* DNA polymerase from NEB, *Taq* DNA polymerase from Roche, *Taq* DNA polymerase from Qiagen, KlenTaq 1 from DNA Polymerase Technology and Bst DNA polymerase large fragment from NEB.

According to an embodiment, the method further comprises (b-5) determining the amount of the nucleic acid polymerase with activity contained in the product obtained from the preparation process from the mass of the protein contained in the product and the purity of the nucleic acid polymerase contained in the product provided in step (b-4).

According to a more specific embodiment of the present invention, the measurement of the specific activity of the reference nucleic acid polymerase in step (b-3) is carried out by a method comprising the following steps: (b-3-1) adding the reference nucleic acid polymerase to an composition for activity assay of a nucleic acid polymerase and performing a nucleic acid polymerization reaction to detect a signal; wherein the composition for activity assay of a nucleic acid polymerase comprises a primer, the primer is extended by the reference nucleic acid polymerase under polymerization conditions to form an extended duplex, and a detectable signal is provided in a manner dependent on the extended duplex; (b-3-2) providing the activity of the reference nucleic acid polymerase from the intensity of the signal; and (b-3-3) providing the activity of the reference nucleic acid polymerase relative to the mass of the reference nucleic acid polymerase as the specific activity of the reference nucleic acid polymerase.

According to an embodiment, the method further includes the following steps after step (b):

(b-5) obtaining a product from a preparation process different from the preparation process of the nucleic acid polymerase of step (a); wherein the product comprises a protein containing the nucleic acid polymerase,
(b-6) performing step (b) using the product obtained from the preparation process of step (b-5) to provide the specific activity of nucleic acid polymerase contained in a product obtained from the preparation process of step (b-5); and
(b-7) comparing the specific activity of nucleic acid polymerase contained in a product obtained from the preparation process of step (a) with the activity of nucleic acid polymerase contained in a product obtained from the preparation process of step (b-6).

Step (c): providing the nucleic acid polymerase (230)

Finally, the method of the present invention comprises (c) providing the nucleic acid polymerase in which the specific activity of the nucleic acid polymerase contained in the product obtained from the preparation process has been measured.

In this step, the nucleic acid polymerase is provided as a final purified product in which the state of the nucleic acid polymerase contained in the product obtained from the preparation process of a nucleic acid polymerase is evaluated.

According to an embodiment of the present invention, the nucleic acid polymerase is a nucleic acid polymerase in which the purity of nucleic acid polymerase contained in a product obtained from the preparation process is calculated.

## EFFECTS OF THE INVENTION

The features and advantages of the present invention are summarized as follows:

(a) In order to secure the quality of nucleic acid polymerase, which is a diagnostic enzyme, monitoring of the state (activity, purity) of the enzyme in the preparation process (culture and purification process) is important, but the product obtained from the preparation process contains impurities and proteins in an inactive state, and thus there was a problem in that it is very difficult to measure or analyze the activity of nucleic acid polymerase.
(b) The present invention can evaluate the state of a nucleic acid polymerase contained in a product obtained from the preparation process of a nucleic acid polymerase by diluting the product obtained from the preparation process of a nucleic acid polymerase with a dilution factor at which the specific activity of the nucleic acid polymerase contained in the product indicates a substantially constant value.
(c) In addition, the present invention enables measurement of the specific activity of nucleic acid polymerase contained in the product without exchange of a base (or blank) solution of the product obtained from the preparation process through such dilution, and also enables analysis of purity.
(d) The present invention can analyze the state of the nucleic acid polymerase in the preparation process, thereby enabling quality control in the production process of nucleic acid polymerase and contributing to the improvement of quality.

## BRIEF DESCRIPTION OF THE DRAWINGS

FIG. 1 is a flowchart of processes of performing a method of the present invention according to an embodiment of the present invention.

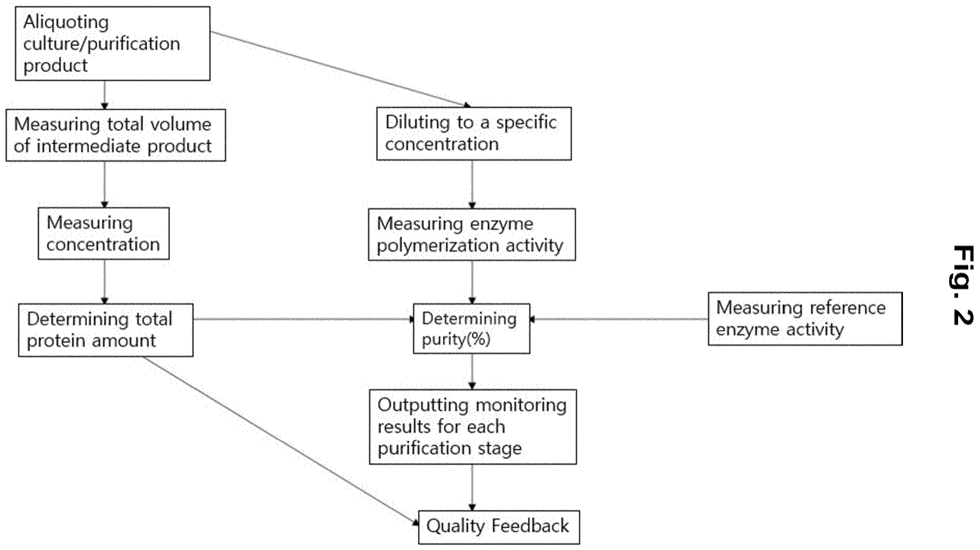

FIG. 2 shows an analysis flowchart according to an embodiment of the present invention.
FIG. 3 shows a real-time fluorescence measurement graph for measuring polymerization activity according to an embodiment of the present invention.
FIG. 4 is a graph of changes in specific activity according to dilution factors of culture and purification intermediate products according to an embodiment of the present invention.

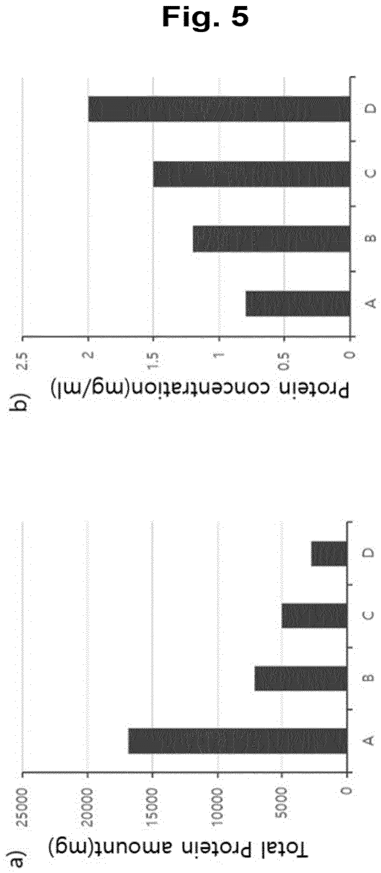

FIG. 5 shows a graph measuring the total amount and concentration of proteins of intermediate products for culture and purification according to an embodiment of the present invention.

FIG. 6 shows a graph showing the result of calculating the enzyme specific activity of an intermediate product of culture and purification according to an embodiment of the present invention.

FIG. 7 is a graph showing the purity of an intermediate product of culture and purification according to an embodiment of the present invention.

FIG. 8 is a flowchart of processes of performing a method of the present invention according to another embodiment of the present invention.

The present invention will now be described in further detail by examples. It would be obvious to those skilled in the art that these examples are intended to be more concretely illustrative and the scope of the present invention as set forth in the appended claims is not limited to or by the examples.

**Examples**

Example 1: Measurement of specific activity of a reference *Taq* DNA polymerase (reference or standard product)

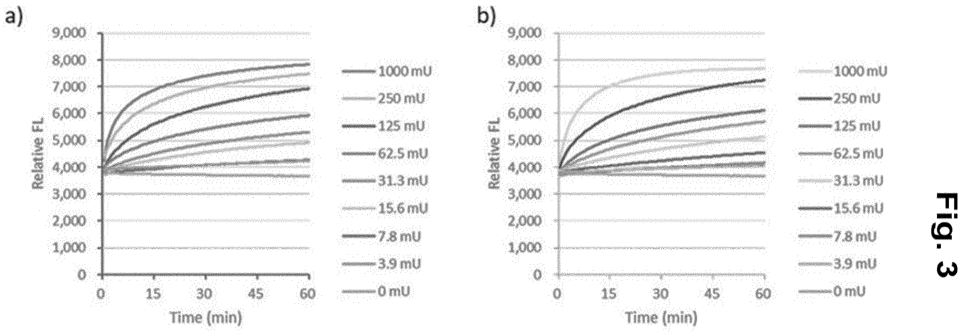

Selection of the reference nucleic acid polymerase (reference or standard product) as measured by the precise measurement criteria is important to identify the precise criteria for the specific activity of the culture and purification intermediate products and to calculate the purity based on them. First, *Taq* DNA polymerase (Source: Native Taq) from NEB Company and *Taq* DNA polymerase (Source: Native Taq) from Roche Company were selected as reference nucleic acid polymerases. The concentration of each enzyme was confirmed in Jesse L. Montgomery et al. Analytical Biochemistry 2013, 441(2), 133-139. In addition, in order to calculate the initial polymerization rate according to the protein dilution factor by using the EvaEZ™ product (including EvaGreen® dye, primed template, dNTPs, $MgCl_2$, and Tris buffer), which is a fluorometric polymerase activity assay kit of Biotium Company, the change of fluorescence for 60 minutes at 30 sec intervals was tracked using CFX96-DR equipment of Bio-Rad (see, (a) and (b) of FIG. 3), and information on the error range was accurately confirmed through three repetitions within the same set for the same experiment, *i.e.,* a total of seven sets repetitions.

The process of selecting a *Taq* DNA polymerase from NEB Company and *a Taq* DNA polymerase from Roche Company as standard products (reference nucleic acid polymerases) and measuring their specific activities is as follows:

1) The preparation of a reagent for the selection of a standard product

For the selection of the standard product, 5 U/ul of *Taq* DNA polymerase of NEB Company, which is being sold, was prepared in concentrations of 1000 mU/ul, 500 mU/ul, 250 mU/ul, 125 mU/ul, 62.5 mU/ul, 31.3 mU/ul, 15.6 mU/ul, 7.8 mU/ul, and 3.9 mU/ul using 10X Thermopol reaction buffer and Ultrapure water of New England Biolabs Company, respectively, and the reagents were prepared by mixing them at volume ratios of 10:1:9 (EvaEZ reagent, enzyme, and Ultrapure water), and all of these processes were performed in a state of lowering the temperature using ice ((a) of FIG. 3). In addition, a reagent was prepared by diluting 5 U/ul of *Taq* DNA polymerase of Roche Company in the same manner as above ((b) of FIG. 3).

2) reaction of a reagent

In order to observe the kinetic reaction, the reagent of 1) above divided by 20 ul was put into a 8-strip PCR tube in CFX96 of Bio-Rad Company, a temperature-controlled instrument capable of real-time fluorescence measurement, and the instrument was set up and reacted to allow the reaction to be proceeded at 72°C and measure fluorescence at 30-second intervals. The slope of the part with the initial linear function was calculated from the extracted data.

Specifically, in the graph (x-axis time, y-axis fluorescence value) of FIG. 3, the slope (differential value of time-fluorescence value) of a portion whose fluorescence value over time is a first order function is measured within the first 10 minutes for each concentration of the enzyme of 1). Since this value is a value of fluorescence that increases with time when DNA is synthesized, it has a direct correlation with the consumption of dNTP. Then, it was confirmed that the differential value of the time-fluorescence value according to the enzyme concentration of 1) is a first order function, and the slope of the first order function (the differential value of the enzyme concentration-'time-fluorescence value') was calculated and used as an index for the dNTP consumption directly proportional to the enzyme concentration. Thus, the unit for the polymerization activity of the DNA polymerase could be defined as the amount of dNTP consumed per unit time at the unit temperature.

3) the measurement of the specific activity of *Taq* DNA polymerase of 100% purity

Through the process 2), the *Taq* DNA polymerase contained in the reagent 1) was defined as a standard product when a constant value was calculated every time through the process of changing the instrument, changing the experimenter, and changing the reagent to be used. These standard products were buffer exchanged and concentrated through amicon tubes (Amicon Ultra centrifugal filter units, 30 kDa cut-off) and then subjected to SDS-PAGE analysis. Standard materials for Bovine Serum Albumin (BSA) quantification were loaded together in the same SDS-PAGE in the same volume from 0.1 mg/ml to 1 mg/ml for each concentration, and the specific activity value was calculated after quantifying the amount of enzyme in the actual standard product based on this data.

In conclusion, it was confirmed that the definition of 1 Unit for the *Taq* DNA polymerase from NEB Company, measured on the basis of radioisotopes, that is, the level of consuming 15 nmole of dNTP for 30 minutes at 75°C, was the same as the level of consuming 4.4±0.56 nmole (95% confidence interval) of dNTP for 30 minutes at 72°C of the *Taq* DNA polymerase from NEB Company using the EvaEZ™ product, thereby securing reliability for the change in the measurement method of the reference *Taq* DNA polymerase (standard product).

In addition, it was confirmed that the definition of 1 Unit for Roche's *Taq* DNA polymerase measured on the basis of radioisotopes, that is, the level of consuming 10 nmole of dNTP for 30 minutes at 75°C, was the same as the level of consuming 4.4±0.56 nmole (95% confidence interval) of dNTP for 30 minutes at 72°C of the *Taq* DNA polymerase from Roche Company using EvaEZ™ product, thereby confirming that the same confidence level could be measured for Roche's product (reference *Taq* DNA polymerase).

In addition, the specific activity of *Taq* DNA polymerase, which is a reference nucleic acid polymerase of 100% purity, was calculated using EvaEZ™ products and used in the examples described below.

Example 2: determination of dilution factor in culture and purification intermediate product analysis

The gene capable of expressing the target protein was cloned and transformed into *E. coli* to obtain a culture product containing *Taq* DNA polymerase.

Then, the culture product including the *Taq* DNA polymerase was heat-treated and subjected to two purification processes in total of ion exchange column purification and metal exchange column purification to obtain the heat treatment product A, the eluate B of the ion exchange column purification, the eluate C of the metal bond column purification and the final purification product D.

Since each culture and purification intermediate product has a different eluate base (or blank) solution, the effect on the measurement of enzyme activity may be different, so whether a constant activity value can be measured regardless of the type of eluate was confirmed through dilution of the culture and purification intermediate product. The specific process is as follows:

1) preparation of a heat treatment product A (intermediate product immediately after culture)

Transgenic *E. coli* (transformed with plasmids containing *Taq* DNA polymerase and His Tag) cells cultured at 37°C were separated from the broth solution via centrifuge, remelted with buffer (20 mM Tris, pH 8, 500 mM NaCl, 0.1% Triton X-100), and broken the cell membrane down by sonication while incubated in ice to elute the proteins. These samples were separated from the sinking part and the remaining part in the solution using a centrifuge of 13,000 rpm, and only the remaining part in the solution was obtained. To further remove impurities from the corresponding part, the subsided part was removed by centrifuge again after treatment at 80°C for 60 min. The supernatant at this time was used as the heat treatment product A.

2) preparation of eluate B of ion exchange column purification

The heat-treated product A was first filtered through a syringe filter of 0.2 micrometers, and then subjected to a purification process through an anion exchange column (HiTrap Q of Cytiva) using FPLC equipment (AKTA pure of Cytiva). Proteins were separated and purified by increasing the concentration of KCl with 20 mM phosphate buffer in the anion exchange column. The fraction that passed through the FPLC of each purification product was analyzed by SDS-PAGE to select a section with a high concentration of the target protein. The fractions selected in this way were collected into one tube and used as eluate B for ion exchange column purification.

3) preparation of eluate C of metal binding column purification

In order to additionally purify the eluate B of the ion exchange column purification, a His taq purification column using nickel (HisTrap of Cytiva) was mounted on a FPLC (AKTA pure of Cytiva) equipment in a metal exchange column and used the column. Since the enzyme was designed to have His Tag, the enzyme was bound to this column, and impurities were flowed through a 20 mM phosphate buffer, and then the concentration of imidazole was raised to 500 mM to separate only the desired enzyme into a specific fraction. As in 2) above, each fraction was analyzed by SDS-PAGE, and the fraction portion from which the desired protein was eluted was selected, collected into one tube, and used as the eluate C of the metal binding column purification.

4) preparation of final purified product D

The eluate C solution of the metal binding column purification was filtered through a 0.2 micrometer syringe filter, and proteins (including target enzymes) of a filter unit or more were concentrated using a Amicon tube (Amicon Ultra centrifugal filter units, 30 kDa cut-off), and small proteins of the size of the target protein or less were further removed. This resulted in the preparation of the final purification product D.

5) experiment determining dilution factor

Since it is not possible to obtain reliability of analysis of the intermediate products of culture and purification only by the concentration of protein in the state in which the purity of the intermediate products of culture and purification A to C is not known, it was confirmed that the specific activity could be measured by simultaneously diluting each solution by a specific dilution factor. All dilutions were made to a final 1 x using NEB's 10x Thermopol reaction buffer. The dilution of all three solutions of A, B, and C was increased by 1-fold to 2-fold, they were diluted to 1 in 65536 and the specific activity (U/ug) was measured by the method of Example 1 to confirm the dilution factor in which all three solutions maintained significant values.

FIG. 4 shows changes in specific activity according to the dilution factor of the intermediate products of the culture and purification, the heat treatment product A, the eluate B of the ion exchange column purification, and the eluate C of the metal bond column purification.

As a result, interestingly, it was found that a constant activity value could be measured regardless of the type of eluate at a specific dilution concentration interval. Specifically, regardless of the type of eluate, significant activity data could be analyzed regardless of the amount of impurity at a dilution factor ranging from 60 to 2000 times (protein concentration in the range of 0.0005-0.0125 mg/ml).

Example 3: analysis of intermediate products of culture and purification

In the same manner as in Example 2, a heat treatment product A, an eluate B of ion exchange column purification, and an eluate C of metal bond column purification, which is an intermediate product of culture and purification including *Taq DNA* polymerase, were obtained, and then the final purified product D was obtained.

The concentration of each purified product was determined using the Bradford method, and the total amount of protein was obtained through the volume of the total eluate, and the results are shown in (a) and (b) of FIG. 4.

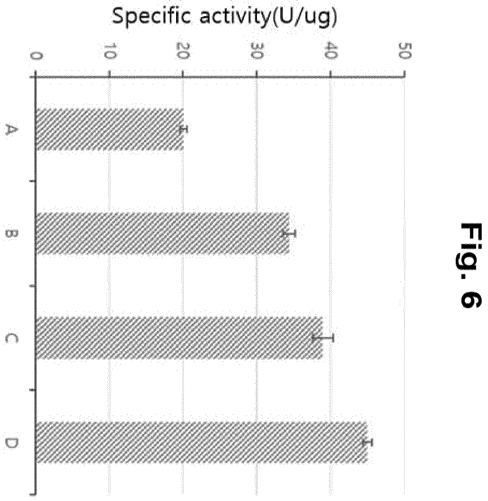

In addition, the heat treatment product A, the eluate B of the ion exchange column purification, the eluate C of the metal binding column purification, and the final purification product D were diluted in the range of 60 to 2000 times identified in Example 2 to obtain polymerase activity according to the dilution factor using EvaEZ™, and the specific activity was calculated by dividing the polymerase activity by a protein concentration value and summarized in FIG. 6.

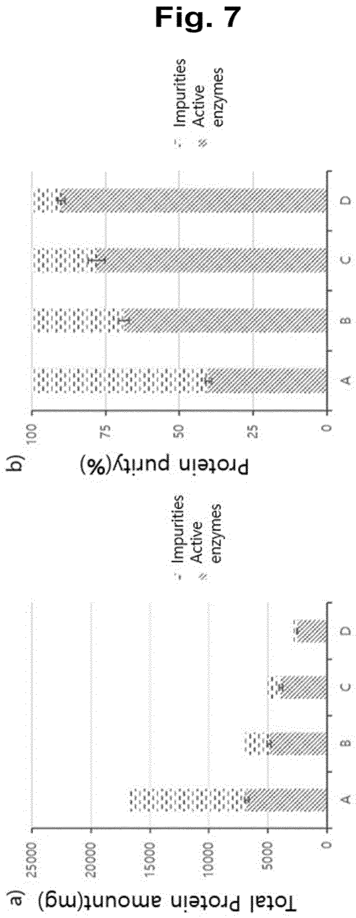

In order to calculate the purity of the culture and purification intermediate product through the calculated specific activity value and the given total protein amount, the specific activity value of the reference *Taq* DNA polymerase (reference product) identified through Example 1 was used. Based on the specific activity value identified through the process of Example 1, the amount of enzyme having activity among all enzymes could be calculated by Equation ([total protein amount] x [specific activity]/[specific activity of the reference product]), and the results are summarized in (a) of FIG. 7. In addition, based on this, the content of impurities during each purification process was calculated as a ratio, and is shown in (b) of FIG. 7.

In conclusion, in the case of the present embodiment, it was confirmed that the impurities were well removed according to the purification order, and finally, an enzyme having high purity and activity was produced.

Having described a preferred embodiment of the present invention, it is to be understood that variants and modifications thereof falling within the spirit of the invention may become apparent to those skilled in this art, and the scope of this invention is to be determined by appended claims and their equivalents.

**Claims**

1. A method for evaluating the state of a nucleic acid polymerase contained in a product obtained from a preparation process of the nucleic acid polymerase, comprising:

(a) obtaining the product from the preparation process of the nucleic acid polymerase; wherein the product comprises a protein containing the nucleic acid polymerase, and
(b) diluting the product obtained from the preparation process to a dilution factor at which specific activity of the nucleic acid polymerase contained in the product shows a substantially constant value and measuring the specific activity of the nucleic acid polymerase by using the diluted product; wherein the specific activity is measured from intensity of a signal measured by adding the diluted product to a composition for activity assay of a nucleic acid polymerase and performing nucleic acid polymerization reaction, the composition for activity assay of a nucleic acid polymerase comprises a primer, the primer is extended by the nucleic acid polymerase contained in the product under polymerization condition to form an extended duplex, and a detectable signal is provided in a manner dependent on the extended duplex.

2. A method for obtaining a QC-able nucleic acid polymerase from a product obtained from a preparation process of the nucleic acid polymerase, comprising:

(a) obtaining the product from the preparation process of the nucleic acid polymerase; wherein the product comprises a protein containing the nucleic acid polymerase,
(b) diluting the product obtained from the preparation process to a dilution factor at which specific activity of the nucleic acid polymerase contained in the product shows a substantially constant value and measuring the specific activity of the nucleic acid polymerase by using the diluted product; wherein the specific activity is measured from intensity of a signal measured by adding the diluted product to a composition for activity assay of a nucleic acid polymerase and performing nucleic acid polymerization reaction, the composition for activity assay of a nucleic acid polymerase comprises a primer, the primer is extended by the nucleic acid polymerase contained in the product under polymerization condition to form an extended duplex, and a detectable signal is provided in a manner dependent on the extended duplex, and
(c) providing the nucleic acid polymerase in which the specific activity of the nucleic acid polymerase contained in the product obtained from the preparation process has been measured.

3. The method according to claim 1 or 2, wherein the nucleic acid polymerase is a DNA polymerase.

4. The method according to claim 3, wherein the DNA polymerase is *Taq* DNA polymerase, KlenTaq DNA polymerase, Klenow fragment of *E. coli* DNA polymerase I, DNA polymerase I, T4 DNA polymerase, Bst DNA polymerase, Bsu DNA polymerase, MMLV reverse transcriptase, AMV reverse transcriptase or HIV reverse transcriptase.

5. The method according to claim 1 or 2, wherein the preparation process of the nucleic acid polymerase is carried out by a method comprising the following steps:

(a-1) culturing host cells containing an expression construct for a nucleic acid polymerase; and
(a-2) purifying the nucleic acid polymerase by applying the resultant of the step (a-1) to a column chromatography.

6. The method according to claim 1 or 2, wherein the method further comprises, after step (a), (a-3) determining mass of the protein contained in the product obtained from the preparation process from (i) volume of the product obtained from the preparation process of step (a) and (ii) concentration of the protein in the product.

7. The method according to claim 1 or 2, wherein the dilution in step (b) is performed within a range of 60 to 2000 times.

8. The method according to claim 1 or 2, wherein the product is at least one product selected from the group consisting of a host cell lysate, a heat treatment product of host cell lysate, and a resultant of column chromatography.

9. The method according to claim 1 or 2, wherein the dilution in step (b) is performed using a buffer.

10. The method according to claim 9, wherein the buffer comprises Tris, Cl$^-$ and Mg$^{2+}$, and satisfies pH 7 to pH 9.

11. The method according to claim 1 or 2, wherein the concentration of the protein contained in the diluted product in step (b) is 0.0005 mg/ml to 0.0125 mg/ml.

12. The method according to claim 1 or 2, wherein the measurement of the specific activity in step (b) is carried out by a method comprising the following steps:

(b-1) diluting the product obtained from the preparation process of step (a) to a dilution factor at which specific activity of the nucleic acid polymerase contained in the product shows a substantially constant value and measuring activity of the nucleic acid polymerase by using the diluted product; wherein the activity is measured from intensity of a signal measured by adding the diluted product to a composition for activity assay of a nucleic acid polymerase and performing nucleic acid polymerization reaction, the composition for activity assay of a nucleic acid polymerase comprises a primer, the primer is extended by the nucleic acid polymerase contained in the product under polymerization condition to form an extended duplex, and a detectable signal is provided in a manner dependent on the extended duplex, and

(b-2) providing the activity of the nucleic acid polymerase contained in the product measured in step (b-1) relative to the mass of the protein contained in the product as the specific activity of the nucleic acid polymerase contained in the product obtained from the preparation process.

13. The method according to claim 1 or 2, wherein the composition for activity assay of a nucleic acid polymerase further comprises (i) a template, (ii) a label, (iii) dNTP and (iv) a buffer.

14. The method according to claim 1 or 2, wherein in step (b), the addition of the diluted product to a composition for activity assay of a nucleic acid polymerase is performed by adding the diluted product to a composition for activity assay of a nucleic acid polymerase by one dilution factor or by each dilution factor within the range of the dilution factor.

15. The method according to claim 1 or 2, wherein the nucleic acid polymerization reaction is carried out at 35°C to 75°C for 10 to 60 minutes.

16. The method according to claim 13, wherein the label is an intercalating label.

17. The method according to claim 16, wherein the intercalating label is selected from the group consisting of SYBR™ Green I, PO-PRO™-1, BO-PRO™-1, SYTO™ 43, SYTO™ 44, SYTO™ 45, SYTOX™ Blue, POPO™-1, POPO™-3, BOBO™-1, BOBO™-3, LO-PRO™-1, JO-PRO™-1, YO-PRO™ 1, TO-PRO™1, SYTO™11, SYTO™13, SYTO™15, SYTO™16, SYTO™20, SYTO™23, TOTO™-3, YOYO™3, GelStar™, thiazole orange, PicoGreen®, EvaGreen® and LAM Fluorescent Dye.

18. The method according to claim 12, wherein the activity of the nucleic acid polymerase in step (b-1) is provided as a slope measured in a range where the intensity of a signal increases as a linear function depending on the concentration of the protein contained in the product obtained from the preparation process.

19. The method according to claim 12, wherein the nucleic acid polymerase is *Taq* DNA polymerase or KlenTaq DNA polymerase, and the activity of the nucleic acid polymerase in step (b-1) is provided as the amount of a nucleic acid polymerase determined by substituting the intensity of a signal into the following general formula 1:

**General formula 1** 1 unit (enzyme activity unit) = the amount of nucleic acid polymerase required to represent intensity of a signal showing polymerization by consuming $4.4 \pm 0.56$ nmole of dNTP for 30 minutes at 72°C.          General formula 1

20. The method according to claim 12, wherein the nucleic acid polymerase is *Taq* DNA polymerase or KlenTaq DNA polymerase, and the activity of the nucleic acid polymerase in step (b-1) is provided as the unit number of a nucleic acid polymerase determined by substituting the intensity of a signal into the following general formula 1:

**General formula 1** 1 unit (enzyme activity unit) = the amount of nucleic acid polymerase required to represent intensity of a signal showing polymerization by consuming $4.4 \pm 0.56$ nmole of dNTP for 30 minutes at 72°C.          General formula 1

21. The method according to claim 1 or 2, wherein the method further comprises, after step (b), the following steps:

(b-3) providing specific activity of a reference nucleic acid polymerase; and

(b-4) providing the specific activity of the nucleic acid polymerase provided in step (b) relative to the specific activity of the reference nucleic acid polymerase in step (b-3) as purity of the nucleic acid polymerase contained in the product obtained from the preparation process.

22. The method according to claim 21, wherein the method further comprises (b-5) determining the amount of the nucleic acid polymerase with activity contained in the product obtained from the preparation process from the mass of the protein contained in the product and the purity of the nucleic acid polymerase contained in the product provided in step (b-4).

23. The method according to claim 21, wherein the reference nucleic acid polymerase is a nucleic acid polymerase with known activity and purity.

# Fig. 1

*100*

*110* — obtaining the product from the preparation process of the nucleic acid polymerase

*120* — diluting the product obtained from the preparation process to a dilution factor at which specific activity of the nucleic acid polymerase contained in the product shows a substantially constant value and measuring the specific activity of the nucleic acid polymerase by using the diluted product

# Fig. 2

# Fig. 3

# Fig. 4

# Fig. 5

# Fig. 6

# Fig. 7

# Fig. 8

*200*

*210*
┌─────────────────────────────────────┐
│ obtaining the product from the       │
│ preparation process of the nucleic acid │
│ polymerase                           │
└─────────────────────────────────────┘

*220*
┌─────────────────────────────────────┐
│ diluting the product obtained from the │
│ preparation process to a dilution factor │
│ at which specific activity of the nucleic │
│ acid polymerase contained in the     │
│ product shows a substantially constant │
│ value and measuring the specific activity │
│ of the nucleic acid polymerase by using │
│ the diluted product                  │
└─────────────────────────────────────┘

*230*
┌─────────────────────────────────────┐
│ providing the nucleic acid polymerase in │
│ which the specific activity of the nucleic │
│ acid polymerase contained in the     │
│ product obtained from the preparation │
│ process has been measured            │
└─────────────────────────────────────┘

## INTERNATIONAL SEARCH REPORT

| International application No. |
| --- |
| **PCT/KR2024/001370** |

### A. CLASSIFICATION OF SUBJECT MATTER

**C12Q 1/48**(2006.01)i; **C12Q 1/686**(2018.01)i

According to International Patent Classification (IPC) or to both national classification and IPC

### B. FIELDS SEARCHED

Minimum documentation searched (classification system followed by classification symbols)

C12Q 1/48(2006.01); C12Q 1/68(2006.01); C12Q 1/686(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 핵산 중합효소(polymerase), 고유활성(specific activity), 희석(dilution), 프라이머 (primer), 평가(evaluation)

### C. DOCUMENTS CONSIDERED TO BE RELEVANT

| Category* | Citation of document, with indication, where appropriate, of the relevant passages | Relevant to claim No. |
| --- | --- | --- |
| A | KR 10-2380264 B1 (SEEGENE, INC.) 29 March 2022 (2022-03-29)<br>See claims 1 and 2. | 1-23 |
| A | CN 114032285 B (BEIJING QINGKE BIOTECHNOLOGY CO., LTD. et al.) 24 May 2022 (2022-05-24)<br>See claims 1 and 7. | 1-23 |
| A | CN 112176040 A (MARINE RESOURCES DEVELOPMENT INSTITUTE OF JIANGSU (LIANYUNGANG) et al.) 05 January 2021 (2021-01-05)<br>See claim 1. | 1-23 |
| A | EP 2256215 A1 (MERGEMEIER, Steffen) 01 December 2010 (2010-12-01)<br>See claims 1 and 3. | 1-23 |
| A | WO 2010-036359 A2 (ALLELOGIC BIOSCIENCES CORPORATION) 01 April 2010 (2010-04-01)<br>See claim 1. | 1-23 |

☐ Further documents are listed in the continuation of Box C.   ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **20 May 2024** | **22 May 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2024/001370**

| Patent document cited in search report | | | Publication date (day/month/year) | Patent family member(s) | | | Publication date (day/month/year) |
|---|---|---|---|---|---|---|---|
| KR | 10-2380264 | B1 | 29 March 2022 | EP | 3676400 | A2 | 08 July 2020 |
| | | | | KR | 10-2020-0052905 | A | 15 May 2020 |
| | | | | US | 11591645 | B2 | 28 February 2023 |
| | | | | US | 2021-0071230 | A1 | 11 March 2021 |
| | | | | WO | 2019-045532 | A2 | 07 March 2019 |
| | | | | WO | 2019-045532 | A3 | 31 May 2019 |
| CN | 114032285 | B | 24 May 2022 | CN | 114032285 | A | 11 February 2022 |
| CN | 112176040 | A | 05 January 2021 | CN | 112176040 | B | 17 November 2023 |
| EP | 2256215 | A1 | 01 December 2010 | EP | 2256216 | A1 | 01 December 2010 |
| WO | 2010-036359 | A2 | 01 April 2010 | WO | 2010-036359 | A3 | 17 June 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20230023613

**Non-patent literature cited in the description**

- **CHIEN A.** ; **EDGAR D. B.** ; **TRELA J. M.** *J. Bacteriol.*, 1976, vol. 127, 1550-1557
- **CHARLES C.RICHARDSON** ; **CARL L.SCHILDK-RAUT** ; **H. VASKEN APOSHIAN** ; **ARTHUR KORN-BERG**. *J. Biol. Chem.*, 1964, vol. 239 (1), 222-232
- **MARK A.GRIEP**. *Anal. Biochem.*, 1995, vol. 232, 180-189
- **LIMING YU** ; **GUOLU HU** ; **LEIGHTON HOWELLS**. *Biotechniques*, 2002, vol. 33, 938-941
- **H TVEIT** ; **T KRISTENSEN**. *Anal. Biochem.*, 2001, vol. 289, 96-98
- **FATEME MOAZEN** ; **ALI RASTEGARI** ; **SAYED MEHDI HOSEINI** ; **MOJTABA PANJEHPOUR** ; **MEHRAN MIROLIAEI** ; **HAMID MIR** ; **MOHAMMAD SADEGHI**. *Adv Biomed Res.*, 2012, vol. 1 (2), 82
- **SCHEIT**. Nucleotide Analogs. John Wiley, 1980
- **UHLMAN** ; **PEYMAN**. *Chemical Reviews*, 1990, vol. 90, 543-584
- **SAMBROOK et al.** Molecular Cloning, A Laboratory Manual. Cold Spring Harbor Laboratory Press, 2001
- **YANOFSKY, C.** *J. Bacteriol.*, 1984, vol. 158, 1018-1024
- **HERSKOWITZ, I.** ; **HAGEN, D.** *Ann. Rev. Genet.*, 1980, vol. 14, 399-445
- *Appl. Environ. Microbiol.*, 1998, vol. 64, 3932-3938
- *Mol. Gen. Genet.*, 1996, vol. 250, 734-741
- **SCHEK, N et al.** *Mol. Cell Biol.*, 1992, vol. 12, 5386-5393
- **KLASENS, B. I. F. et al.** *Nucleic Acids Res.*, 1998, vol. 26, 1870-1876
- **GIL, A. et al.** *Cell*, 1987, vol. 49, 399-406
- **COLE, C. N.** ; **T. P. STACY**. *Mol. Cell. Biol.*, 1985, vol. 5, 2104-2113
- **BATT, D. B** ; **G. G. CARMICHAEL**. *Mol. Cell. Biol.*, 1995, vol. 15, 4783-4790
- **CAPECCHI, M.R.** *Cell*, 1980, vol. 22, 479
- **HARLAND** ; **WEINTRAUB**. *J. Cell Biol.*, 1985, vol. 101, 1094-1099
- **GRAHAM, F.L. et al.** *Virology*, 1973, vol. 52, 456
- **CHEN** ; **OKAYAMA**. *Mol. Cell. Biol.*, 1987, vol. 7, 2745-2752
- **NEUMANN, E. et al.** *EMBO J.*, 1982, vol. 1, 841
- **TURKASPA et al.** *Mol. Cell Biol.*, 1986, vol. 6, 716-718
- **WONG, T.K. et al.** *Gene*, 1980, vol. 10, 87
- **NICOLAU** ; **SENE**. *Biochim. Biophys. Acta*, 1982, vol. 721, 185-190
- **NICOLAU et al.** *Methods Enzymol.*, 1987, vol. 149, 157-176
- **GOPAL**. *Mol. Cell Biol.*, 1985, vol. 5, 1188-1190
- **YANG et al.** *Proc. Natl. Acad. Sci.*, 1990, vol. 87, 9568-9572
- **JOSEPH SAMBROOK et al.** Molecular Cloning, A Laboratory Manual. Cold Spring Harbor Laboratory Press, 2001
- **M.L.M. ANDERSON**. Nucleic Acid Hybridization. Springer-Verlag New York Inc., 1999
- **JESSE L. MONTGOMERY et al.** *Analytical Biochemistry*, 2013, vol. 441 (2), 133-139